# FASCICULE DE BREVET EUROPEEN

(11) **EP 1 089 677 B1**
(45) Date de publication et mention de la délivrance du brevet: **06.05.2004**
(21) Numéro de dépôt: 99957143.3
(22) Date de dépôt: 22.06.1999
(51) Int. Cl.: A61F 2/44

(54) **IMPLANT INTERSOMATIQUE RACHIDIEN A ELEMENTS D'ANCRAGES**
INTERSOMATISCHES WIRBELSÄULENIMPLANTAT MIT VERANKERUNGSELEMENTEN
BACKBONE INTERSOMATIC IMPLANT WITH ANCHORING ELEMENTS

(30) Priorité: 23.06.1998 FR 9807909; 17.06.1999 FR 9907669
(43) Date de publication de la demande: 11.04.2001
(73) Titulaire: Dimso Distribution Medicale du Sud Ouest, 33610 Cestas (FR)
(72) Inventeur: CROZET, Yves, F-74600 Seynod (FR); LE COUEDIC, Régis, F-33610 Cestas (FR); GAUCHET, Fabien, Route de Rocquemont, 60800 Duvy (FR); PASQUET, Denis, F-33000 Bordeaux (FR)
(74) Mandataire: Texier, Christian
(86) Numéro de dépôt international: PCT/FR1999/001492
(87) Numéro de publication internationale: WO 1999/066867

(56) Documents cités:
- EP-A- 0 260 044
- EP-A- 0 637 439
- EP-A- 0 697 200
- WO-A-97/06753
- WO-A-97/48352
- FR-A- 2 717 068
- FR-A- 2 727 003
- US-A- 5 702 391

## Description

L'invention concerne les implants intersomatiques rachidiens.

On connaît d'après le document FR-2 727 003 un implant intersomatique rachidien destiné à être disposé à la place d'un disque intervertébral après ablation, et comprenant un corps présentant deux faces planes de contact avec les corps vertébraux adjacents. Il comporte deux logements de réception de vis d'ancrage, disposés de sorte que les vis s'étendent en saillie des faces de contact respectives pour être ancrées dans les corps vertébraux adjacents. Chaque vis est inclinée par rapport à la face de contact associée du fait que la tête de vis s'étend à un bord du corps pour pouvoir être manoeuvrée une fois l'implant reçu entre les corps vertébraux. Toutefois, la mise en place des vis est difficile à effectuer du fait de l'inclinaison de leur axe. De plus, le positionnement des vis pour optimiser la qualité de l'ancrage ne peut être amélioré sans nuire à leur accessibilité.

On connaît par ailleurs du document US-5 702 391 un implant intersomatique comprenant un corps, des picots mobiles à coulissement dans le corps pour s'étendre en saillie de faces externes du corps et des cames sphériques mobiles à coulissement dans un conduit axial du corps. Une pièce d'actionnement disposée à l'embouchure du conduit permet de pousser les cames, ce qui déplace par effet de rampe les picots pour les mettre en saillie et ainsi ancrer l'implant dans les plateaux vertébraux des vertèbres associées. Un tel implant rend beaucoup plus facile d'effectuer l'ancrage robuste entre les vertèbres. Cependant, cet implant est très difficile à ôter le cas échéant, ce qui en revanche n'était pas le cas avec l'implant du document FR-2 727 003 précité puisqu'il suffisait de dévisser les vis pour supprimer l'ancrage de l'implant dans les plateaux.

Un but de l'invention est de fournir un implant pouvant facilement être installé et ôté.

En vue de la réalisation de ce but, on prévoit selon l'invention un implant intersomatique rachidien comportant un corps, au moins un élément d'ancrage mobile par rapport au corps pour s'étendre en saillie d'une face de contact du corps avec une vertèbre, et au moins une came coulissante par rapport au corps et apte à déplacer l'élément d'ancrage par rapport au corps par effet de rampe avec l'élément d'ancrage, dans lequel la came et l'élément d'ancrage sont agencés pour que la came déplace l'élément d'ancrage dans deux sens de déplacement opposés.

Ainsi, on déplace l'élément d'ancrage par l'intermédiaire de la came en agissant sur cette dernière. Puisqu'on n'agit plus directement sur l'élément d'ancrage, on s'affranchit dans une très large mesure des contraintes liées à l'accessibilité de l'élément d'ancrage. Dès lors, la manoeuvre de ce dernier est plus facile à effectuer lors d'une intervention. De plus, puisqu'il n'est plus nécessaire de rendre l'élément d'ancrage directement accessible, on peut modifier son positionnement de très nombreuses façons pour qu'il assure sa fonction d'ancrage le mieux possible. Par conséquent, on facilite la manoeuvre pour l'installation de l'élément d'ancrage tout en permettant d'améliorer la qualité de l'ancrage.

Par ailleurs, l'action de la came étant réversible, elle permet d'actionner le ou chaque élément d'ancrage pour le faire passer de la position étendue à la position rétractée. Il est ensuite facile d'ôter l'implant. Cette action de la came sur l'élément d'ancrage est positive en ce sens que la came entraîne l'élément d'ancrage. L'action de la came ne consiste pas seulement à laisser la voie libre à l'élément d'ancrage pour qu'il puisse rentrer dans le corps sous l'effet d'une sollicitation externe telle que la pression exercée sur l'élément d'ancrage par le matériau de la vertèbre. On peut donc notamment ôter l'implant longtemps après sa mise en place.

Des modes de réalisation sont présentés dans les revendications dépendantes.

D'autres caractéristiques et avantages de l'invention apparaîtront encore dans la description suivante de quatre modes préférés de réalisation donnés à titre d'exemples non limitatifs. Aux dessins annexés :
- les figures 1 et 2 sont deux vues en perspective respectivement à l'état monté et éclaté d'un implant qui ne concerne pas la présente invention ;
- les figures 3 et 4 sont deux vues en coupe de l'implant de la figure 1 durant deux étapes respectives de son installation ;
- la figure 5 est une vue analogue à la figure 4 montrant l'implant entre deux vertèbres ;
- les figures 6 et 7 sont deux vues respectivement en perspective éclatée et en coupe d'un implant selon un premier mode de réalisation de l'invention ;
- la figure 8 est une vue en perspective d'un implant selon un deuxième mode de réalisation de l'invention ;
- la figure 9 est une vue en perspective de l'implant de la figure 8 avec la partie supérieure du corps ôtée;
- les figures 10 et 11 sont des vues en perspective respectivement à l'état monté et éclaté d'un implant selon un troisième mode de réalisation de l'invention ;
- la figure 12 est une vue partielle en coupe axiale de la came et de la vis de la figure 11 ;
- la figure 13 est une vue en perspective d'un implant selon le quatrième mode de réalisation de l'invention ;
- la figure 14 est une vue en coupe de l'implant suivant le plan XIV-XIV de la figure 13 ;
- les figures 15 et 16 sont deux vues en perspective de la came et des porte-éléments de l'implant de la figure 13 respectivement en position rétractée et en position étendue des éléments ; et
- la figure 17 est une vue en coupe de la came et des porte-éléments suivant le plan médian XVII-XVII de la figure 16.

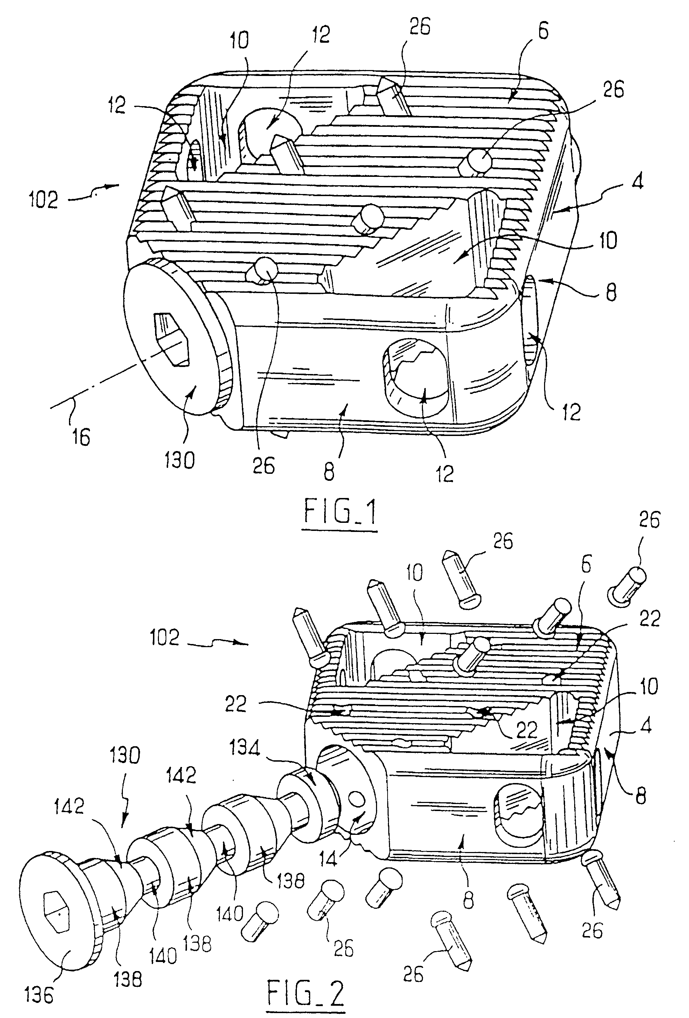

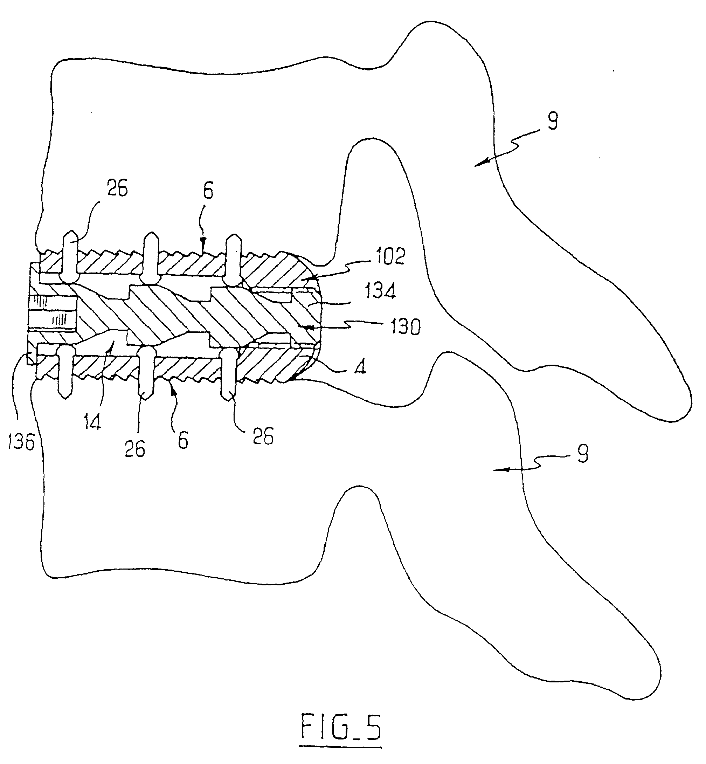

On va décrire un implant en référence aux figures 1 à 5. L'implant 102 comprend un corps 4 en forme générale de parallélépipède rectangle aplati suivant une dimension de façon à définir deux grandes faces supérieure et inférieure 6 et quatre faces latérales 8. Les arêtes et les coins du corps sont arrondis pour ne pas blesser les tissus du corps humain auquel l'implant est destiné. Les faces supérieure et inférieure 6 ont une forme générale plane et un profil transversal en dents de scie ou zigzag, les sommets des dents définissant des arêtes continues parallèles entre elles. Ces arêtes assurent une bonne prise des faces supérieure et inférieure 6 sur les plateaux des corps vertébraux des vertèbres 9 de destination, comme le montre la figure 5.

Le corps 4 présente deux évidements traversants 10 s'étendant à proximité de deux arêtes opposées du corps et de l'une à l'autre des faces supérieure et inférieure 6 en débouchant sur celles-ci. De plus, chaque face latérale 8 présente un orifice 12 débouchant dans l'un des évidements 10 à mi-distance des deux faces supérieure et inférieure 6. Lors de la mise en place de l'implant, les évidements 10 sont remplis de greffon qui pourra ainsi croître en direction des plateaux vertébraux à travers les évidements 10 et les orifices 12.

Le corps 4 présente un conduit cylindrique 14 s'étendant avec son axe 16 de l'une à l'autre de deux arêtes du corps opposées l'une à l'autre et distantes des évidements 10, parallèlement à un plan général des faces supérieure et inférieure 6. Le conduit 14 débouche à ses deux extrémités. Au voisinage de l'une distale des extrémités, il présente un tronçon 18 de diamètre rétréci et fileté. Le reste du conduit 14 constitue un tronçon 20 lisse et de plus grand diamètre par comparaison avec le tronçon 18.

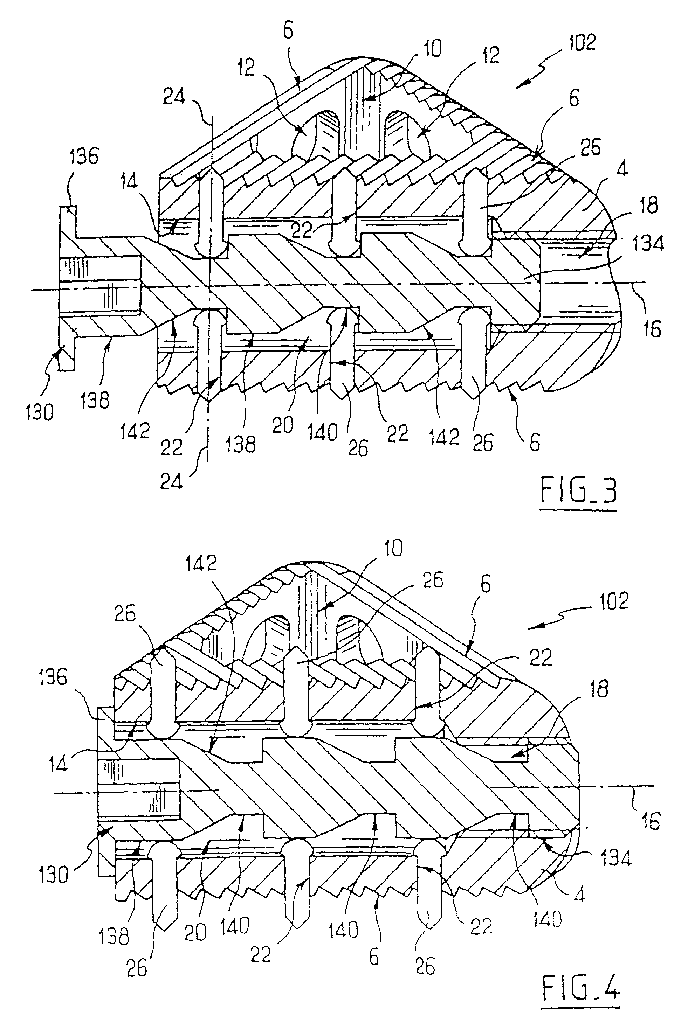

Le corps 4 présente des canaux 22 de forme cylindrique s'étendant depuis le tronçon 20 jusqu'aux faces supérieure et inférieure 6. Tous les canaux 22 ont un axe 24 qui coupe l'axe 16 du tronçon 20, ici à angle droit. Les canaux 22 sont ici au nombre de douze. Ils sont répartis en deux groupes de six canaux 22. Dans chaque groupe, les six canaux 22 sont parallèles entre eux, coaxiaux par paires, et situés dans un même plan incluant l'axe 16. C'est dans un tel plan qu'a été effectuée la coupe des figures 3 et 4 de sorte que les six canaux d'un seul groupe sont visibles sur ces figures. Ce plan est incliné par rapport au plan général des faces supérieure et inférieure 6 de sorte que les axes 24 des canaux 22 le sont aussi. Les inclinaisons sont symétriques et de même valeur pour les deux groupes de canaux. Dans chaque groupe de six canaux 22, trois débouchent sur la face supérieure 6 et trois sur la face inférieure 6. Chaque canal 22 qui débouche dans la face supérieure 6 est coaxial avec l'un de ceux du même groupe qui débouche sur la face inférieure. Dans chaque groupe, les trois canaux 22 qui débouchent sur une même face sont espacés d'un même pas. Ainsi sur chaque face 6, débouchent des canaux 22 répartis en deux rangées définissant des directions d'alignement parallèles entre elles, les canaux de chaque rangée ayant des inclinaisons opposées.

Chaque canal 22 reçoit, mobile à coulissement dans le canal, un élément d'ancrage qui est ici un picot 26 ayant un corps cylindrique lisse présentant à une extrémité la plus proche de la face 6 une pointe et à l'autre extrémité une tête à face sphérique convexe de plus grand rayon que le rayon du canal 22 et de plus grande largeur que le diamètre du canal 22 associé. La tête est située dans le tronçon 20 et retient ainsi le picot 26 prisonnier à l'encontre de sa sortie totale par la face 6 associée.

L'implant comprend une came 130 de forme de révolution autour d'un axe coaxial à l'axe 16. Elle présente à une extrémité axiale distale une face cylindrique filetée 134 apte à former une liaison vis-écrou avec le tronçon 18 du corps. A une extrémité axiale proximale 136, elle présente une tête de plus grand diamètre que le diamètre du tronçon 20 du corps pour venir en butée extérieurement contre le corps. La tête présente une empreinte, par exemple hexagonale, à six pans, pour permettre de mettre la came 130 en rotation autour de son axe au moyen d'un outil adapté tel qu'une clé.

Entre ces deux extrémités, la came 130 présente trois faces cylindriques larges 138, trois faces cylindriques 140 étroites par référence aux faces larges 138, et trois faces tronconiques 142 inclinées en direction de l'extrémité distale filetée 134. Ces faces sont alternées et réparties en trois groupes consécutifs comprenant chacun dans le sens proximal-distal une face large 138, une face tronconique 142 et une face étroite 140, la face tronconique 142 constituant une transition de niveau entre les deux autres faces. Les longueurs des faces selon l'axe 16 sont identiques pour chaque type de face. Ces longueurs sont adaptées de sorte que, en référence à la figure 3, lorsque l'extrémité distale filetée 134 de la came est en prise avec seulement l'extrémité proximale du tronçon 18, les têtes des picots 26 sont en appui sur les faces étroites 140, chaque face étroite 140 étant en contact avec les têtes de quatre picots 26 dont les axes 24 sont coplanaires dans un plan perpendiculaire à l'axe 16, alors qu'en référence à la figure 4, lorsque la tête de la came 130 est en butée sur le corps, les têtes des picots 26 sont en appui sur les faces larges 138, chaque face large 138 étant en contact avec les quatre picots 26 précités. Le diamètre des faces étroites 140 est tel que dans la position de la figure 3, que nous appellerons "position rentrée", la pointe des picots 26 ne s'étend pas en saillie de la face 6 associée, ou s'étend si modérément en saillie qu'elle n'entrave pas significativement l'installation de l'implant 102 entre les corps vertébraux. Le diamètre des faces larges 138 est tel qu'en position de la figure 4, que nous appellerons "position sortie", les picots 26 s'étendent en saillie de la face 6, par exemple sur le quart ou le tiers de leur longueur, et pénètrent dans le corps vertébral associé suffisamment pour interdire le retrait de l'implant.

La mise en place de l'implant est effectuée de la façon suivante. Après ablation d'un disque intervertébral, et après avoir rempli les évidements 10 avec du greffon comme précité, on introduit l'implant 102 entre les corps vertébraux des vertèbres 9 associées au disque ôté. La hauteur du corps 4 de l'implant correspond à l'épaisseur du disque ôté. L'implant est introduit de sorte que le tronçon fileté 18 est sensiblement en position postérieure. Les faces 6 s'étendent en regard des plateaux vertébraux respectifs, parallèlement à ceux-ci et en contact avec ceux-ci. L'implant est introduit dans la configuration rentrée de la figure 3.

Une fois l'implant à son emplacement, on manoeuvre à l'aide d'une clé la tête de la came 130 située en position antérieure pour faire tourner la came autour de son axe 16. Compte tenu de la liaison vis-écrou entre l'extrémité distale 134 de la came et le tronçon 18, la came suit donc une trajectoire hélicoïdale suivant son axe 16. Pour chaque groupe de quatre picots, la face tronconique contiguë 142 vient progressivement en contact avec les têtes des picots et constitue une rampe qui, compte tenu de son déplacement vers le tronçon 18, sollicite les quatre picots pour les faire coulisser vers l'extérieur du corps. Au fur et à mesure de leur extension en saillie, les picots 26 pénètrent dans les plateaux vertébraux pour ancrer l'implant dans les vertèbres. Les quatre picots 26 viennent ensuite en appui sur la face large contiguë 138 et s'étendent en saillie des faces 6 respectives, en configuration sortie. A la fin de la manoeuvre de la came 130, la tête de la came est en appui contre le corps et son extrémité distale 134 est à l'extrémité distale du tronçon 18.

Dans une variante, la liaison vis-écrou entre la came 130 et le corps 4 pourra être remplacée par un encliquetage ou clipsage immobilisant la came par rapport au corps à l'issue d'une simple poussée sur la came parallèlement à son axe. La came est alors mobile à coulissement par rapport au corps. Elle pourra alors avoir transversalement à son axe une section non circulaire, par exemple rectangulaire.

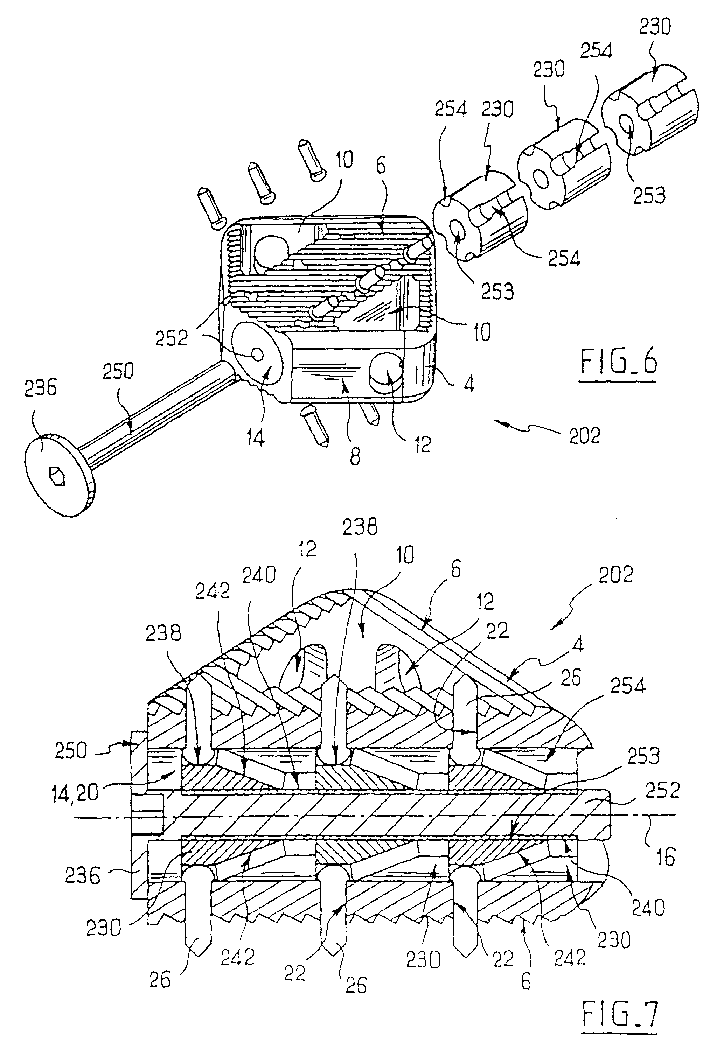

On va maintenant décrire un premier mode de réalisation de l'implant selon l'invention en référence aux figures 6 et 7. Les références des éléments différents de ceux du premier mode ont été augmentées de 100.

Dans l'implant 202, le corps 4 a sensiblement la même configuration que dans le précédant, mis à part que le tronçon large lisse 20 constitue la totalité du conduit 14 de came. La came 130 est ici remplacée par un ensemble de trois cames 230 et d'une vis 250. La vis 250 a une tête de manoeuvre 236 formant butée sur le corps, analogue à celle de la came 130. La vis a une tige filetée 252.

Les trois cames 230 sont identiques entre elles. Chaque came 230 a une forme générale cylindrique. Elle présente un conduit cylindrique fileté 253 apte à coopérer avec la tige 252 suivant une liaison vis-écrou. Chaque came 230 présente quatre gorges 254 associées respectivement en propre à l'un des quatre picots 26 destinés à être actionnés par la came. Chaque gorge 254 s'étend dans un plan radial à l'axe 16 de la came. Chaque gorge 254 présente une partie haute peu profonde 238, une partie basse 240 profonde par rapport à la partie haute, et une partie intermédiaire 242 formant transition de niveau entre les parties haute et basse. Les parties hautes des quatre gorges 254 d'une même came sont contiguës à l'extrémité proximale de cette came. Chaque gorge a, perpendiculairement à l'axe 16, un profil en arc de cercle ouvert vers l'extérieur et s'étendant sur plus d'un demi cercle, de rayon constant le long de la gorge, les bords de l'arc de cercle étant prolongés vers l'extérieur au niveau des parties intermédiaires et basses par deux flancs plans parallèles entre eux. Chaque gorge est adaptée à recevoir suivant la direction axiale par chaque extrémité la tête du picot 26 associé, en interdisant la sortie de la tête suivant la direction radiale à la came. Le fond de chaque gorge 254 constitue une première rampe faire coulisser le picot 26 associé vers l'extérieur lorsque la came coulisse vers l'extrémité distale du conduit 14. Un intérêt de ce mode de réalisation est qu'il est réversible. En effet, la tête de chaque picot 26 étant prisonnière dans la gorge associée 254, les bords de cette gorge constituent une deuxième rampe qui permet de replacer le picot en position rentrée lorsque la came 254 coulisse vers l'extrémité proximale du canal.

Le montage des différentes pièces de l'implant est effectué comme suit. Après réception des picots 26 dans leurs conduits 22 du corps 4, on fait coulisser l'une des cames 230 jusqu'à la position centrale associée aux picots 26 du milieu de la rangée. Pour cela, la came 230 doit "franchir" les quatre picots 26 de l'une des extrémités des rangées, par exemple les quatre picots 26 de l'extrémité distale si la came est introduite par cette extrémité. Cette étape est obtenue en introduisant la came 232 dans l'extrémité distale du conduit 14 en insérant les picots 26 dans les gorges 254 respectives. Les picots 26 étant initialement en saillie, on continue de pousser la came vers le centre du conduit ce qui, compte tenu des effets de rampe par les gorges, place les picots 26 en configuration rentrée. Pendant qu'on continue à pousser la came, les picots sortent par l'extrémité distale des gorges en partie basse 240. On achève de pousser la came pour introduire les picots du centre des rangées dans les gorges 254 en partie haute 238 et les faire arriver finalement en partie basse 240 de la came. On introduit ensuite de même la came 230 à placer en position distale. Puis on introduit de même par l'extrémité proximale la came 230 destinée à occuper la position la plus proximale. Tous les picots 26 étant en partie basse 240 des gorges, en configuration rentrée, on visse la vis 250 dans les cames 230 pour la mettre en prise avec celles-ci.

Après installation de l'implant entre les vertèbres, on pousse la vis 250 en direction de l'extrémité distale du conduit 14, ce qui fait coulisser les cames 230 dans le même sens. Par effet de rampe sur le fond des gorges 254, les picots coulissent alors dans leur conduit pour passer de la partie basse 240 à la partie haute et aboutir en position sortie. Compte tenu de la réversibilité précitée, au moyen d'étapes inverses de celles mises en oeuvre pour le montage, on peut donc ôter l'implant de son emplacement entre les vertèbres.

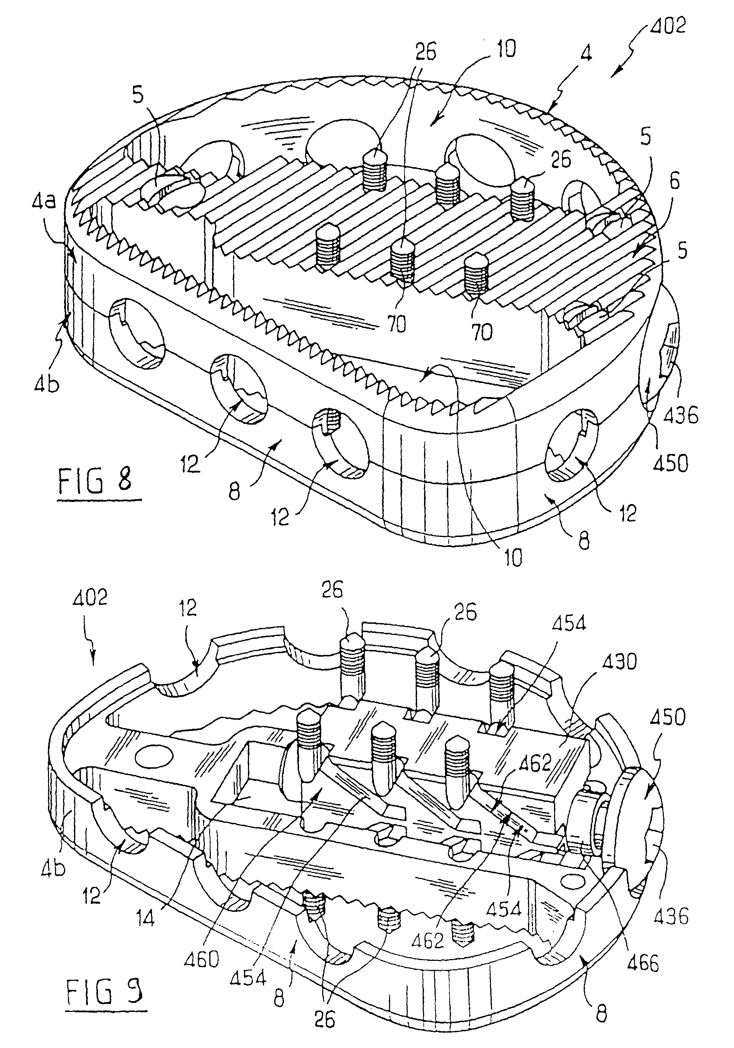

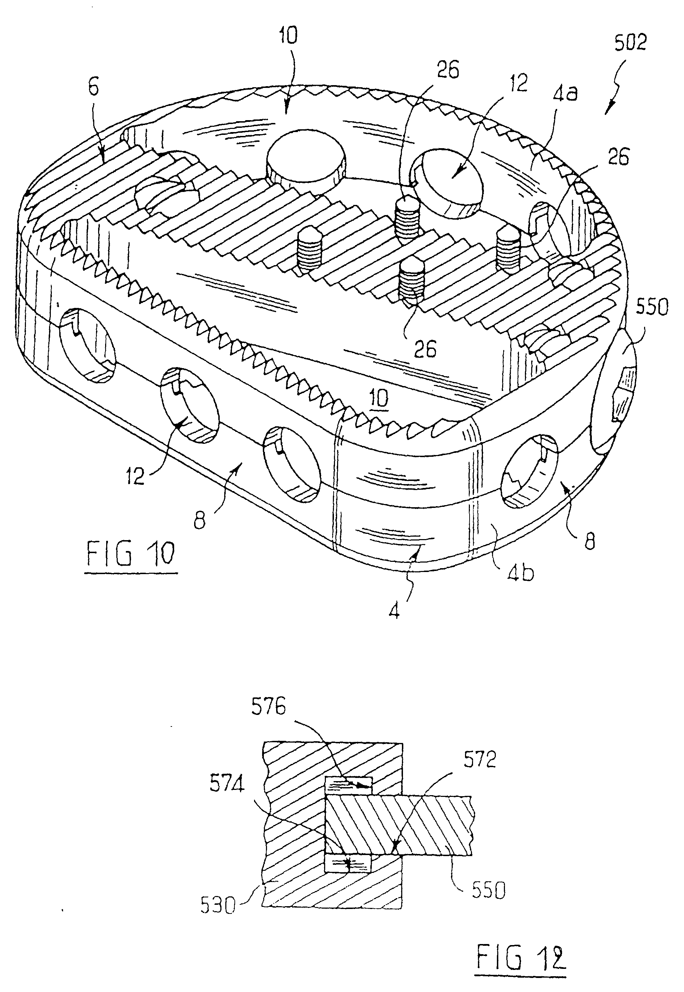

Dans le deuxième mode de réalisation, illustré aux figures 8 et 9 avec certaines références augmentées de 300, le corps 4 a en plan une forme générale en demi-disque, limité par une paroi latérale plane 8 destinée à être en position postérieure et une paroi latérale circulaire 8 destinée à être en position antérieure. Chaque paroi latérale 8 présente des orifices 12 débouchant dans les évidements 10 comme précité. Le corps 4 comprend cette fois deux parties supérieure 4a et inférieure 4b se rejoignant suivant un plan de joint parallèle aux faces supérieure et inférieure 6 portant chacune l'une de ces faces 6, et fixées l'une à l'autre au moyen de vis 5. Les conduits 22 sont encore au nombre de douze mais ils sont orientés avec leurs axes perpendiculaires aux faces supérieure et inférieure 6.

Le conduit de came 14 a une section de forme généralement rectangulaire perpendiculairement à son axe 16 et est défini pour moitié par chaque partie 4a, 4b du corps. La came 430 a une section rectangulaire mâle correspondant à celle femelle du conduit 14 la recevant. Elle est apte à y coulisser suivant son axe. La came a une forme générale de parallélépipède rectangle. Les deux faces latérales longitudinales 460 de la came 430 présentent chacune six gorges 454 aptes à recevoir respectivement les picots 26 en propre. A cette fin, les picots présentent chacun en lieu et place de leur tête une excroissance reçue dans la gorge, orientée en direction de l'autre rangée de picots, et leur donnant une forme générale de "L". Chaque gorge 454 présente deux faces ou rampes planes 462, parallèles entre elles, perpendiculaires à la face latérale 460 associée, et inclinée par rapport à la direction de coulissement de sorte que l'extrémité de la gorge 454 la plus proche de la face 6 associée est la plus éloignée de l'extrémité proximale de la came.

La came 430 présente un conduit fileté la traversant suivant son axe. L'implant 402 comprend une vis 450 présentant une tige filetée apte à coopérer en liaison filetée avec la came 430. La vis 450 présente une gorge recevant un collier 466 solidaire du corps 4, de sorte que la vis 450 est montée mobile à rotation dans le corps tandis que la came 430 est mobile à coulissement dans le corps.

Après installation de l'implant 402 entre les vertèbres 9 avec les picots 26 en position rentrée, lorsqu'on manoeuvre la tête 436 de la vis, la rotation de la vis provoque le coulissement de la came 430 en direction de l'extrémité distale du conduit 14, ici borgne, et par effet de rampe sur les faces 462 des gorges orientées vers la face 6 associée, le coulissement des picots 26 pour leur mise en saillie et en position sortie.

Le fonctionnement de l'implant 402 est encore réversible, les faces 462 des gorges orientées en direction opposée à la face 6 associée étant aptes à placer les picots 26 en position rentrée lorsque la came 430 coulisse vers l'extrémité proximale du conduit 14. En l'espèce, l'extrémité proximale, associée à la tête de vis 436, débouche sur la partie gauche de la paroi latérale courbe 8. Les picots 26 présentent ici au voisinage de leur pointe, sur leur tronçon destiné à être en saillie, des rainures circonférentielles 70 présentant une face en dépouille par rapport au sens de coulissement des picots pour leur mise en saillie et améliorant leur ancrage en permettant la croissance osseuse dans les rainures.

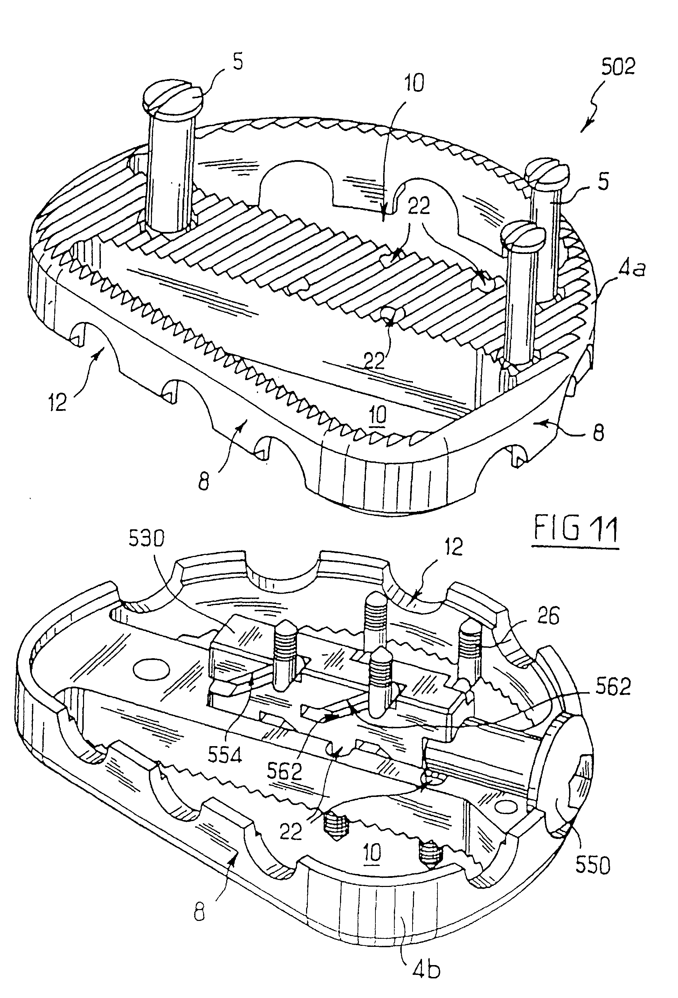

Les figures 10 et 11 présentent un troisième mode de réalisation avec certaines références augmentées de 400. Les picots 26 sont ici au nombre de quatre pour chaque face 6 et sont orientés comme dans le deuxième mode. Les gorges 562 sont analogues à celles du deuxième mode mais sont orientées en sens contraire de sorte que le coulissement de la came 530 vers l'extrémité distale du conduit 14 de came produit la mise en saillie des picots 26. L'implant 502 comporte une vis 550 en liaison vis-écrou avec le corps 4 coaxialement au conduit de came et apte à pousser la came 530 à son extrémité proximale dans son conduit en direction de l'extrémité distale du conduit.

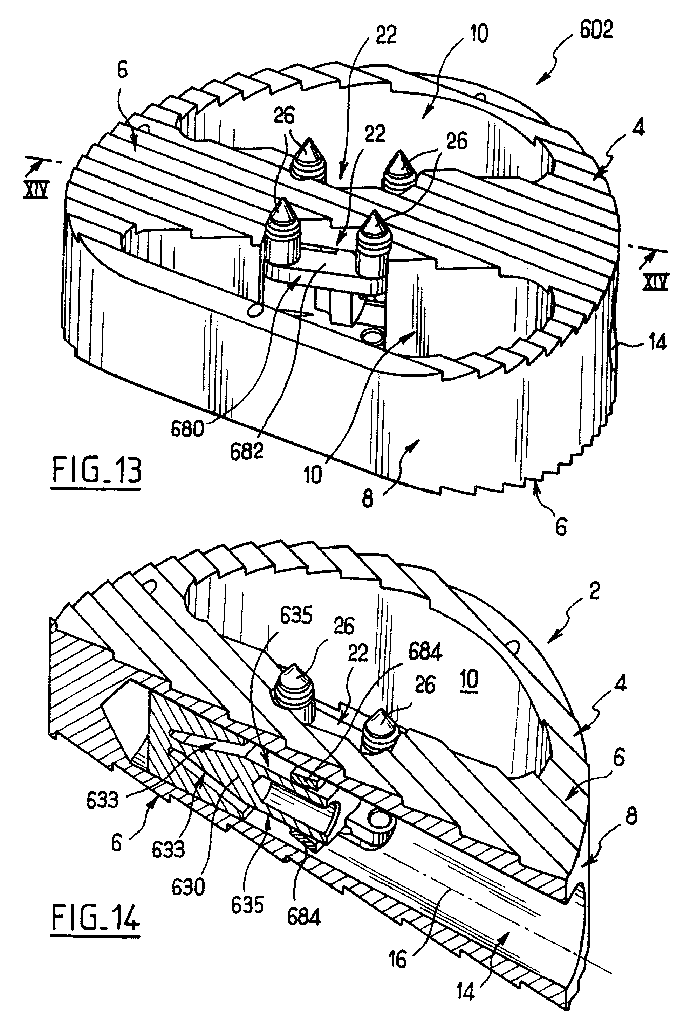

En référence à la figure 12, l'extrémité proximale de la came présente une découpe 572 apte à recevoir à coulissement l'extrémité de la vis 550 pour sa mise en appui sur la came 530. Entre le fond de la découpe et son bord, la came 530 présente une gorge 574 ayant un flanc 576 en dépouille par référence à la direction de coulissement de la came vers l'extrémité proximale du conduit. Cette face 576 est par exemple plane annulaire, perpendiculaire à l'axe 16 de la came et orientée en direction opposée à l'extrémité proximale.

Pour ôter l'implant, on enlève la vis 550, puis on introduit dans le conduit 14 de came un outil apte à venir en appui sur la face 576 en dépouille pour l'accrocher et tirer la came en direction de l'extrémité proximale du conduit, ce qui provoque la mise des picots 26 en configuration rentrée. Ce mode de réalisation évite de réaliser un conduit fileté traversant la came 530 de part en part. Il permet donc de réduire les dimensions de la came 530 et d'augmenter celle des évidements 10 recevant le greffon.

On va décrire un quatrième mode de réalisation de l'implant en référence aux figures 13 à 17. L'implant 602 comprend un corps 4 ayant en plan une forme générale de haricot à hile postérieure, aplati suivant une dimension de façon à définir deux grandes faces supérieure et inférieure 6 et une face latérale périphérique 8. Les faces supérieure et inférieure 6 ont une forme générale plane et un profil transversal en dents de scie ou zigzag, les sommets des dents définissant des arêtes continues parallèles entre elles. Ces arêtes assurent une bonne prise des faces supérieure et inférieure 6 sur les plateaux des corps vertébraux des vertèbres formant l'emplacement de destination.

Le corps 4 présente deux évidements traversants 10 s'étendant de l'une à l'autre des faces supérieure et inférieure 6 en débouchant sur celles-ci. Lors de la mise en place de l'implant, les évidements 10 sont remplis de greffon qui pourra ainsi croître en direction des plateaux vertébraux à travers les évidements 10.

Le corps 4 présente un conduit cylindrique 14 s'étendant avec son axe 16 entre les évidements 10 parallèlement à un plan général des faces supérieure et inférieure 6 en étant séparé de l'unique portion plane de la face latérale 8 par l'un des évidements 10. Le conduit 14 débouche à une seule de ses extrémités.

Le corps 4 présente deux canaux 22 débouchant chacun sur les deux faces supérieure et inférieure. Chaque canal 22 est profilé suivant un axe perpendiculaire aux faces externes et a en section un profil rectangulaire arrondi à ses extrémités. Chaque canal débouche latéralement d'un côté sur toute sa hauteur dans l'un respectif des évidements 10 auquel il est contigu. Par ailleurs, il débouche latéralement du côté opposé par sa partie médiane dans le conduit 14. Les deux canaux 22 s'étendent en regard l'un de l'autre, de part et d'autre du conduit 14. Les canaux 22 mettent ainsi en communication mutuelle les deux évidements 10 et le conduit 14.

Chaque canal 22 reçoit au voisinage de chaque face externe deux éléments d'ancrage sous la forme chacun d'un picot 26 ayant un corps cylindrique lisse présentant à une extrémité la plus proche de la face 6 une pointe. Chaque picot 26 s'étend contre un bord courbe respectif du canal en vue de coulisser contre celui-ci perpendiculairement à la face externe 6 du corps.

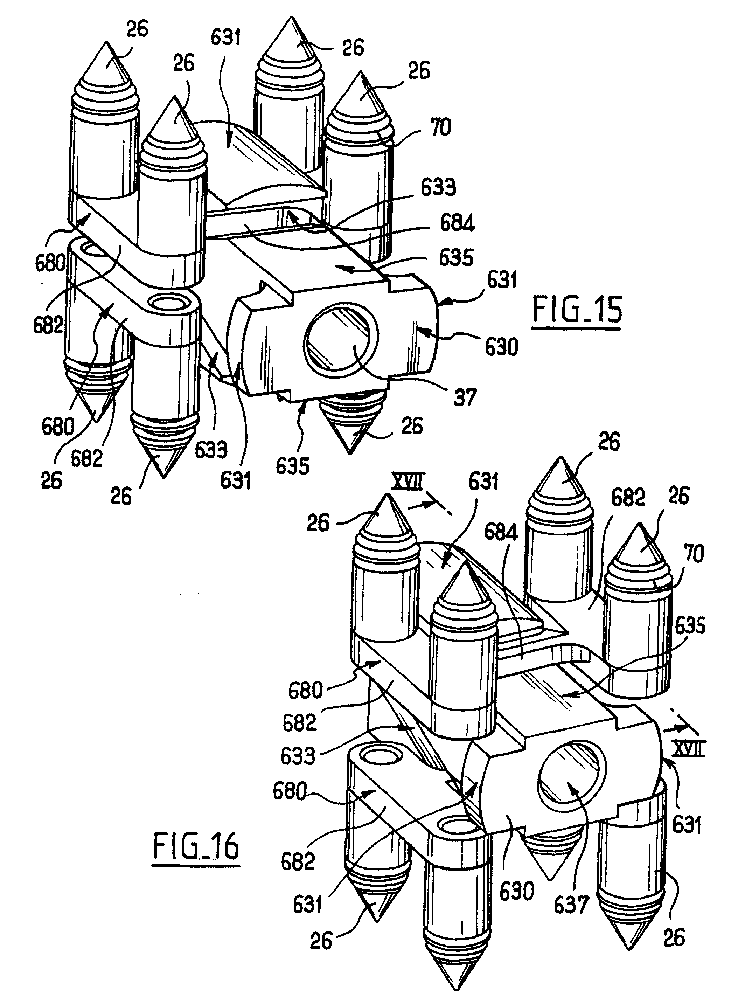

L'implant comporte deux porte-picots ou porte-éléments d'ancrage 680 associés aux faces externes 6 respectives. Chaque porte-picots 80 a une forme générale plate en « H » comprenant deux branches rectilignes 682 parallèles entre elles et un tronçon médian 684 rejoignant les branches à leur milieu. Chaque porte-picots 680 porte rigidement fixé à celui-ci les quatre picots 26 associés à la face externe 6 concernée. Les quatre picots 26 reposent par leur base aux extrémités respectives des branches 682, tous d'un même côté du porte-picots. Les deux porte-picots 680 s'étendent en pérmanence au droit l'un de l'autre avec leurs contours en coïncidence, et cela que les picots 626 soient en position étendue ou rétractée, comme l'illustrent les figures 15 et 16. Les branches 682 s'étendent dans les canaux 622 respectifs dont elles présentent le profil tandis que le tronçon médian 684 s'étend transversalement dans le conduit 14.

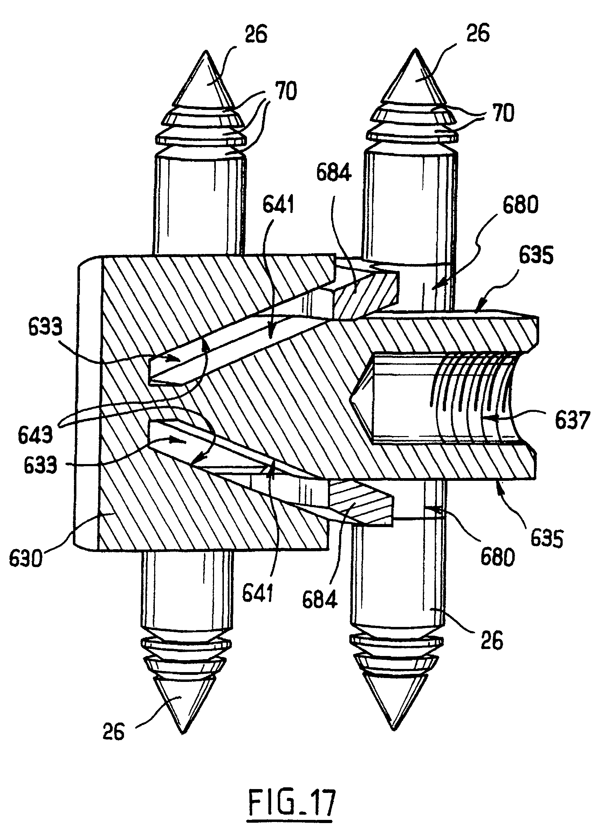

L'implant comporte une came 630 présentant des faces cylindriques 631 gauche et droite à l'arrière et supérieure et inférieure à l'avant pour guider la came à coulissement dans le conduit cylindrique 14. On désigne par arrière de la came son extrémité la plus proche de l'embouchure du conduit 14. Pour chaque porte-picots 680, la came 630 comprend en propre une glissière 633 et une surface d'appui 635 visibles notamment aux figures 14 et 17. La glissière 633 est formée par un conduit très aplati, ouvert sur ses deux bords longitudinaux, incliné par rapport à l'axe 16 en se rapprochant de la face externe 6 associée depuis l'extrémité avant jusqu'à une zone médiane de la came. La glissière 633 est borgne à l'avant et ouverte à l'arrière, la face la plus interne 641 de la glissière étant en continuité avec la surface d'appui 635. Cette dernière est parallèle à l'axe 16 et à la face externe 6 associée. Le tronçon médian 684 peut être manoeuvré par appui sur la glissière 633 et la surface d'appui 635, comme on le verra plus loin. Les glissières inclinées 633 et les surfaces d'appui parallèles 635 donnent à la came une forme d'ancre de marine.

A son extrémité arrière, la came présente un conduit taraudé 637 dont le filet peut être mis en prise avec celui d'un outil adapté afin de manoeuvrer la came pour la pousser ou la tirer.

L'implant est utilisé de la façon suivante, les picots 26 étant initialement en position rétractée, comme sur la figure 15. Après ablation d'un disque intervertébral, et après avoir rempli les évidements 10 avec du greffon comme précité, on introduit l'implant 2 entre les corps vertébraux des vertèbres associées au disque ôté. La hauteur du corps 4 de l'implant correspond sensiblement à l'épaisseur du disque ôté. Les faces 6 s'étendent en regard des plateaux vertébraux respectifs, parallèlement à ceux-ci et en contact avec ceux-ci. La came est près de l'embouchure du conduit 14, à l'arrière.

On visse l'outil dans le conduit 637 de la came et on pousse la came 630 vers l'avant. Par effet de rampe de chaque tronçon médian 684 sur la face la plus interne 641 de la glissière 633 associée, cela entraîne le déplacement, perpendiculairement à la face externe 6, du porte-picots 680 et des quatre picots 26.

Après mise en saillie des picots 26 hors de la face externe 6, la poursuite de la poussée de la came met le tronçon médian 684 en appui sur la surface d'appui 635, comme sur la figure 14, bloquant les picots 26 en position étendue, ancrés dans les plateaux vertébraux. On dévisse alors l'outil pour le séparer de la came.

Si en revanche on souhaite enlever l'implant, on fixe à nouveau l'outil au conduit 637 de la came à travers le conduit 14, puis on tire la came à coulissement vers l'arrière. Le tronçon médian 684 suit alors la surface d'appui 635 puis, par effet de rampe sur la face 643 la plus externe de la glissière 633, il est déplacé vers l'axe 16 vers l'intérieur de l'implant, ce qui rétracte les picots 26 et supprime leur saillie. La came permet donc de manoeuvrer les picots 26 dans les deux sens, c'est-à-dire de façon réversible.

La fixation entre eux des quatre picots 26 de chaque groupe permet d'obtenir un guidage très précis de ceux-ci dans les canaux 22 sans prévoir un canal cylindrique pour chaque picot. De plus, on obtient ce guidage par une seule surface de rampe en deux parties 641, 635 ou 643 pour les quatre picots dans un sens de déplacement donné.

Chaque picot 26 présente à la base de la pointe formant sa tête des rainures de révolution 70 formant des zones en dépouille et améliorant l'ancrage du picot dans le plateau vertébral.

Le corps 4 est formé en deux parties s'assemblant suivant un plan de joint non illustré parallèle aux faces externes 6 et passant par l'axe 16, pour permettre l'introduction de la came 630 et des porte-picots 680 dans le corps.

## Revendications

1. Implant intersomatique rachidien comportant un corps (4), au moins un élément d'ancrage (26) mobile par rapport au corps pour s'étendre en saillie d'une face de contact (6) du corps avec une vertèbre (9), et au moins une came (230 ; 430; 530 ; 630) coulissante par rapport au corps (4) et apte à déplacer l'élément d'ancrage (26) par rapport au corps par effet de rampe avec l'élément d'ancrage, **caractérisé en ce que** la came (230 ; 430 ; 530 ; 630) et l'élément d'ancrage (26) sont agencés pour que la came déplace l'élément d'ancrage (26) dans deux sens de déplacement opposés.

2. Implant selon la revendication 1, **caractérisé en ce que** la came (230 ; 430 ; 630) comporte un actionneur et présente un filet apte à coopérer suivant une liaison vis-écrou avec l'actionneur (250 ; 450) pour manoeuvrer la came depuis l'extérieur du corps (4).

3. Implant selon la revendication 2, **caractérisé en ce que** l'actionneur (450) est apte à être monté mobile à rotation sur le corps (4).

4. Implant selon l'une quelconque des revendications 1 à 3, **caractérisé en ce que** la came (430; 530) est montée mobile à coulissement sur le corps (4).

5. Implant selon l'une quelconque des revendications 1 à 4, **caractérisé en ce que** la came (530) présente une extrémité offrant une face (576) en dépouille par référence à un sens de déplacement de la came pour sa sortie du corps (4).

6. Implant selon l'une quelconque des revendications 1 à 5, **caractérisé en ce qu'**il comporte au moins deux éléments d'ancrage (26) et au moins deux cames (230) aptes à déplacer les éléments d'ancrage respectifs.

7. Implant selon la revendication 6 et l'une quelconque des revendications 2 à 3, **caractérisé en ce que** les deux cames (230) sont agencées pour que leurs filets coopèrent avec un actionneur commun (250) de l'implant.

8. Implant selon l'une quelconque des revendications 1 à 7, **caractérisé en ce que** l'élément d'ancrage (26) est incliné par rapport à un plan général de la face de contact (6).

9. Implant selon l'une quelconque des revendications 1 à 8, **caractérisé en ce qu'**il comporte au moins deux éléments d'ancrage (26) aptes à s'étendre en saillie de la même face de contact (6).

10. Implant selon la revendication 9, **caractérisé en ce que** les éléments d'ancrage (26) sont mobiles à coulissment par rapport au corps suivant des directions respectives non parallèles l'une à l'autre.

11. Implant selon l'une quelconque des revendications 9 à 10, **caractérisé en ce que** la face de contact présente des orifices, chaque orifice recevant un unique élément d'ancrage (26) parmi les éléments d'ancrage respectifs.

12. Implant selon l'une quelconque des revendications 1 à 11, **caractérisé en ce qu'**il comporte au moins quatre éléments d'ancrage (26) aptes à s'étendre en saillie de la même face de contact (6) et disposés suivant deux rangées définissant des directions d'alignement parallèles entre elles.

13. Implant selon l'une quelconque des revendications 1 à 12, **caractérisé en ce que** le corps (4) présente deux faces de contact (6) avec une vertèbre (9) et au moins un évidement (10) s'étendant de l'une à l'autre des faces (6).

14. Implant selon l'une quelconque des revendications 1 à 13, **caractérisé en ce qu'**une partie de l'élément d'ancrage (26) apte à s'étendre en saillie de la face de contact (6) présente des faces (70) en dépouille par rapport à un sens de coulissement de l'élément (26) en direction de la vertèbre (9).

15. Implant selon l'une quelconque des revendications 1 à 14, **caractérisé en ce qu'**il présente au moins deux éléments d'ancrage (26) et un porte-éléments (680) relié rigidement aux éléments d'ancrage.

16. Implant selon la revendication 15, **caractérisé en ce que** la came (630) actionne directement le porte-éléments (680).

17. Implant selon l'une quelconque des revendications 1 à 14, **caractérisé en ce qu'**il comporte au moins deux éléments d'ancrage aptes à être déplacés par la came (230 ; 430 ; 530 ; 630).

18. Implant selon la revendication 17, **caractérisé en ce que** les éléments d'ancrage constituent des pièces distinctes l'une de l'autre et sont chacun en contact direct avec la came (230 ; 430 ; 530).

19. Implant selon l'une quelconque des revendications 1 à 18, **caractérisé en ce que** la came (430 ; 530 ; 630) est unique.

20. Implant selon l'une quelconque des revendications 1 à 19, **caractérisé en ce qu'**il comporte au moins deux éléments d'ancrage (26) aptes à s'étendre en saillie de deux faces respectives opposées du corps.

21. Implant selon l'une quelconque des revendications 1 à 20, **caractérisé en ce que** la ou les faces du corps sont essentiellement planes.

## Patentansprüche

1. Intersomatisches Wirbelsäulenimplantat, umfassend einen Körper (4), wenigstens ein Verankerungselement (26), das bezüglich des Körpers beweglich ist, um sich vorspringend von einer Kontaktfläche (6) des Körpers mit einem Wirbel (9) zu erstrecken, und wenigstens ein Kurvenstück (230; 430; 530; 630) aufweist, das bezüglich des Körpers (4) verschieblich ist und dazu eingerichtet ist, das Verankerungselement (26) bezüglich des Körpers durch Rampenwirkung mit dem Verankerungselement zu versetzen, **dadurch gekennzeichnet, daß** das Kurvenstück (230; 430; 530; 630) und das Verankerungselement (26) derart ausgebildet sind, daß das Kurvenstück das Verankerungselement (26) in zwei entgegengesetzten Versetzungsrichtungen versetzt.

2. Implantat nach Anspruch 1, **dadurch gekennzeichnet, daß** das Kurvenstück (230; 430; 630) ein Betätigungsglied aufweist und ein Gewinde aufweist, das dazu eingerichtet ist, entsprechend einer Schrauben-/Mutterverbindung mit dem Betätigungsglied (250; 450) zusammenzuwirken, um das Kurvenstück von der Außenseite des Körpers (4) her zu betätigen.

3. Implantat nach Anspruch 2, **dadurch gekennzeichnet, daß** das Betätigungsglied (450) dazu eingerichtet ist, drehbeweglich auf dem Körper (4) montiert zu werden.

4. Implantat nach einem der Ansprüche 1 bis 3, **dadurch gekennzeichnet, daß** das Kurvenstück (430; 530) gleitbeweglich an dem Körper (4) angebracht ist.

5. Implantat nach einem der Ansprüche 1 bis 4, **dadurch gekennzeichnet, daß** das Kurvenstück (530) ein Ende aufweist, das eine Freifläche (576) darbietet, bezogen auf eine Versetzungsrichtung des Kurvenstücks, für seinen Austritt aus dem Körper (4).

6. Implantat nach einem der Ansprüche 1 bis 5, **dadurch gekennzeichnet, daß** es wenigstens zwei Verankerungselemente (26) und wenigstens zwei Kurvenstücke (230) aufweist, die dazu eingerichtet sind, die jeweiligen Verankerungselemente zu versetzen.

7. Implantat nach Anspruch 6 und einem der Ansprüche 2 bis 3, **dadurch gekennzeichnet, daß** die beiden Kurvenstücke (230) dazu ausgebildet sind, daß ihre Gewinde mit einem gemeinsamen Betätigungsglied (250) des Implantats zusammenwirken.

8. Implantat nach einem der Ansprüche 1 bis 7, **dadurch gekennzeichnet, daß** das Verankerungselement (26) bezüglich einer allgemeinen Ebene der Kontaktfläche (6) geneigt ist.

9. Implantat nach einem der Ansprüche 1 bis 8, **dadurch gekennzeichnet, daß** es wenigstens zwei Verankerungselemente (26) aufweist, die dazu eingerichtet sind, sich von der Kontaktfläche (6) vorspringend zu erstrecken.

10. Implantat nach Anspruch 9, **dadurch gekennzeichnet, daß** die Verankerungselemente (26) bezüglich des Körpers in jeweils zueinander nicht parallelen Richtungen verschiebbar sind.

11. Implantat nach einem der Ansprüche 9 bis 10, **dadurch gekennzeichnet, daß** die Kontaktfläche Öffnungen aufweist und jede Öffnung unter den jeweiligen Verankerungselementen ein einziges Verankerungselement (26) aufnimmt.

12. Implantat nach einem der Ansprüche 1 bis 11, **dadurch gekennzeichnet, daß** es wenigstens vier Verankerungselemente (26) aufweist, die dazu eingerichtet sind, sich von der selben Kontaktfläche (6) vorspringend zu erstrecken und in zwei Reihen angeordnet zu sein, die zueinander parallele Ausrichtungsrichtungen definieren.

13. Implantat nach einem der Ansprüche 1 bis 12, **dadurch gekennzeichnet, daß** der Körper (4) zwei Berührungsflächen (6) mit einem Wirbel (9) und wenigstens eine Ausnehmung (10) aufweist, die sich von der einen der Flächen (6) zur anderen erstrecken.

14. Implantat nach einem der Ansprüche 1 bis 13, **dadurch gekennzeichnet, daß** ein Teil des Verankerungselements (26), das (6) dazu eingerichtet ist, sich vorspringend von der Kontaktfläche zu erstrecken, Freiflächen (70) bezüglich einer Verschieberichtung des Elements (26) in Richtung des Wirbels (9) aufweist.

15. Implantat nach einem der Ansprüche 1 bis 14, **dadurch gekennzeichnet, daß** es wenigstens zwei Verankerungselemente (26) und ein Trageelement (680) aufweist, das starr mit den Verankerungselementen verbunden ist.

16. Implantat nach Anspruch 15, **dadurch gekennzeichnet, daß** das Kurvenstück (630) die Trageelemente (680) unmittelbar betätigt.

17. Implantat nach einem der Ansprüche 1 bis 14, **dadurch gekennzeichnet, daß** es wenigstens zwei Verankerungselemente aufweist, die dazu eingerichtet sind, durch das Kurvenstück (230; 430; 530; 630) versetzt zu werden.

18. Implantat nach Anspruch 17, **dadurch gekennzeichnet, daß** die Verankerungselemente Teile bilden, die voneinander gesondert sind und von denen jedes in unmittelbarer Berührung mit dem Kurvenstück (230; 430; 530) steht.

19. Implantat nach einem der Ansprüche 1 bis 18, **dadurch gekennzeichnet, daß** das Kurvenstück (430; 530; 630) ein einzelnes ist.

20. Implantat nach einem der Ansprüche 1 bis 19, **dadurch gekennzeichnet, daß** es wenigstens zwei Verankerungselemente (26) aufweist, die dazu eingerichtet sind, sich vorspringend von zwei jeweils entgegengesetzten Flächen des Körpers zu erstrecken.

21. Implantat nach einem der Ansprüche 1 bis 20, **dadurch gekennzeichnet, daß** die Fläche(n) des Körpers im wesentlichen eben ist bzw. sind.

## Claims

1. An intersomatic spine implant comprising a body (4), at least one anchoring element (26) movable relative to the body to project from a contact face (6) of the body making contact with a vertebra (9), and at least one cam (230; 430; 530; 630) slidable relative to the body (4) and suitable for displacing the anchoring element (26) relative to the body by the effect of a ramp engaging the anchoring element, the implant being **characterized in that** the cam (230; 430; 530; 630) and the anchoring element (26) are arranged so that the cam moves the anchoring element (26) in two opposite displacement directions.

2. An implant according to claim 1, **characterized in that** the cam (230; 430; 630) includes an actuator and has a thread suitable for co-operating by screw engagement with the actuator (250; 450) for driving the cam from outside the body (4).

3. An implant according to claim 2, **characterized in that** the actuator (450) is suitable for being mounted to move in rotation relative to the body (4).

4. An implant according to any one of claims 1 to 3, **characterized in that** the cam (430; 530) is mounted to move sliding relative to the body (4).

5. An implant according to any one of claims 1 to 4, **characterized in that** the cam (530) has an end providing a face (576) that is undercut relative to a travel direction of the cam so as to enable the cam to be extracted from the body (4).

6. An implant according to any one of claims 1 to 5, **characterized in that** it includes at least two anchoring elements (26) and at least two cams (230) suitable for moving respective anchoring elements.

7. An implant according to claim 6, and claim 2 or 3, **characterized in that** the two cams (230) are arranged so that their threads co-operate with a common actuator (250) of the implant.

8. An implant according to any one of claims 1 to 7, **characterized in that** the anchoring element (26) slopes relative to a general plane of the contact face (6).

9. An implant according to any one of claims 1 to 8, **characterized in that** it includes at least two anchoring elements (26) suitable for projecting from the same contact face (6).

10. An implant according to claim 9, **characterized in that** the anchoring elements (26) are slidable relative to the body along respective directions that are not parallel to one other.

11. An implant according to claim 9 or claim 10, **characterized in that** the contact face has orifices, each orifice receiving a single anchoring element (26) selected from amongst the respective anchoring elements.

12. An implant according to any one of claims 1 to 11, **characterized in that** it includes at least four anchoring elements (26) suitable for projecting from the same contact face (6) and disposed in two rows defining mutually-parallel alignment directions.

13. An implant according to any one of claims 1 to 12, **characterized in that** the body (4) has two contact faces (6) for making contact with respective vertebrae (9) and at least one recess (10) extending between the contact faces (6).

14. An implant according to any one of claims 1 to 13, **characterized in that** the portion of the anchoring element (26) suitable for projecting from the contact face (6) has faces (70) that are undercut relative to the sliding direction of the element (26) towards the vertebra (9).

15. An implant according to any one of claims 1 to 14, **characterized in that** it presents at least two anchoring elements (26) and an element-carrier (680) rigidly connected to the anchoring elements.

16. An implant according to claim 15, **characterized in that** the cam (630) directly actuates the element-carrier (680).

17. An implant according to any one of claims 1 to 14, **characterized in that** it includes at least two anchoring elements suitable for being moved by the cam (230; 430; 530; 630).

18. An implant according to claim 17, **characterized in that** the anchoring elements constitute parts that are different from one another, each of which is in direct contact with the cam (230; 430; 530).

19. An implant according to any one of claims 1 to 18, **characterized in that** the cam (430; 530; 630) is a single cam.

20. An implant according to any one of claims 1 to 19, **characterized in that** it includes at least two anchoring elements (26) suitable for projecting from two respective opposite faces of the body.

21. An implant according to any one of claims 1 to 20, **characterized in that** the face(s) of the body is/are essentially plane.
